(19) Europäisches Patentamt
European Patent Office
Office européen des brevets

(11) **EP 4 506 401 A1**

(12) **EUROPEAN PATENT APPLICATION**
published in accordance with Art. 153(4) EPC

(43) Date of publication:
**12.02.2025 Bulletin 2025/07**

(21) Application number: **23784768.6**

(22) Date of filing: **05.04.2023**

(51) International Patent Classification (IPC):
*C08J 11/06* (2006.01)   *B29B 17/02* (2006.01)
*A61F 13/49* (2006.01)   *A61F 13/514* (2006.01)

(52) Cooperative Patent Classification (CPC):
**A61F 13/49; A61F 13/514; B29B 17/02; C08J 11/06**

(86) International application number:
**PCT/JP2023/014079**

(87) International publication number:
**WO 2023/195484 (12.10.2023 Gazette 2023/41)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **06.04.2022   JP 2022063616**

(71) Applicant: **Asahi Kasei Kabushiki Kaisha**
**Tokyo 1000006 (JP)**

(72) Inventors:
• **IKEDA, Shogo**
  **Tokyo 100-0006 (JP)**
• **TOYODA, Keiichi**
  **Tokyo 100-0006 (JP)**
• **MORI, Takamasa**
  **Tokyo 100-0006 (JP)**
• **HITOMI, Ryohei**
  **Tokyo 100-0006 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(54) **EASY-TO-RECYCLE ABSORBENT ARTICLE, AND METHOD FOR MANUFACTURING RECYCLED RAW MATERIAL, FIBER, AND FIBER PRODUCT**

(57)   Provided is an absorbent article having an entire exterior part that is easy to recycle, a method for manufacturing a recycled raw material or a fiber from the absorbent article, and a method for manufacturing a fiber product from fiber. The present invention relates to an easy-to-recycle absorbent article that includes an interior part including a front surface sheet and an absorber, and an exterior part that constitutes parts other than the interior part and includes a rear surface sheet and a gather, the absorbent article being characterized in that the rear surface sheet includes a sheet material containing a thermoplastic resin, and the gather contains thermoplastic elastic yarn. The present invention also relates to a method for manufacturing a pelletized recycled raw material, and a fiber and a fiber product for which said absorbent article is used as a raw material.

Fig. 1

EP 4 506 401 A1

**Description**

FIELD

The present invention relates to an easily recyclable absorbent article, and to a method for producing recycled materials, fibers and fiber products.

BACKGROUND

Techniques for recycling used absorbent articles are known.

PTL 1 discloses a method for efficiently producing, from used sanitary products, ash meeting sanitary product standards, and antibacterial recycled pulp, which can be reused for sanitary products, the method being characterized by including an ozone treatment step in which used sanitary products or pulp fibers are immersed in an ozone-containing aqueous solution to decompose the super-absorbent polymer which is in the used sanitary products or adhering to the pulp fibers, wherein prior to the ozone treatment step, simultaneously with the ozone treatment step or after the ozone treatment step, the used sanitary products or pulp fibers are treated with a cationic antimicrobial agent.

PTL 2 discloses a method for producing recycled products from structural components of used absorbent articles, sorting out and recovering the structural components according to their properties and reutilizing them according to their properties, the method comprising a material separation step in which numerous used absorbent articles are separated at least into different films and absorbent body materials, a film-sorting step in which the different separated films are sorted out into different types of recyclable films according to their filler contents, and a pellet-forming step in which the different types of recyclable films that have been separated are used to form different recycled resin pellets according to their filler contents.

[CITATION LIST]

[PATENT LITERATURE]

  [PTL 1] Japanese Unexamined Patent Publication No. 2016-79525
  [PTL 2] Japanese Unexamined Patent Publication No. 2018-171780

SUMMARY

[TECHNICAL PROBLEM]

In recent years there is increasing demand for reducing carbon dioxide and trash produced by treatment of absorbent article waste, and providing added value to the waste by recycling the waste. An absorbent article is generally composed of interior materials including a front sheet and absorbent body, and exterior materials including a back sheet and a gather, with the mass of the exterior part making up a large percentage of about 30% of the total mass of the absorbent article, and it is therefore desirable for such exterior parts to be recycled.

However, PTL 1 only discloses a method of incineration for heat recovery of exterior parts for solid fuel (RPF), without disclosing any technique for recycling the exterior parts.

Moreover, PTL 2 which discloses a method of sorting and recycling of only the exterior films among the structural components of absorbent materials, does not disclose any technique for recycling without sorting all of the exterior parts.

Thus, absorbent articles whose entire exterior parts can be easily recycled, and a method for recycling their entire exterior parts, have not been known.

In light of the aforementioned problems of the prior art, the object of the invention is to provide absorbent articles whose exterior parts can be easily recycled, a method for producing recycled materials or fibers from the absorbent articles, and a method for producing fiber products from the fibers.

[SOLUTION TO PROBLEM]

The present inventors have completed this invention upon finding, unexpectedly, that the aforementioned problem is solved if all of the main members of the exterior parts of absorbent articles include thermoplastic resins.

Specifically, the present invention provides the following.

[1] An easily recyclable absorbent article comprising:

an interior part containing a front sheet and absorbent body, and
an exterior part constituting the non-interior part and containing a back sheet and a gather, wherein the back sheet includes a sheet material comprising a thermoplastic resin, and
the gather includes thermoplastic elastic yarn.

[2] The easily recyclable absorbent article according to [1] above, wherein the outflow start temperature of the thermoplastic elastic yarn is 160°C to 220°C.

[3] The easily recyclable absorbent article according to [1] or [2] above, wherein the thermoplastic elastic yarn is thermoplastic polyurethane yarn.

[4] The easily recyclable absorbent article according to any one of [1] to [3] above, wherein the exterior part contains a hot-melt adhesive.

[5] The easily recyclable absorbent article according to any one of [1] to [4] above, wherein the exterior part contains a thermoplastic film.

[6] The easily recyclable absorbent article according to any one of [1] to [5] above, wherein the coefficient of variation of the melt viscosity of the exterior part is 0.5 or lower, as obtained by the following procedure:

melt kneading the exterior part to obtain pellets;
allowing the pellets to stand for 30 minutes in the furnace of a capillary rheometer set to 230°C;
measuring the melt viscosity at a shear rate of 1857/sec while holding at 230°C; and
calculating the coefficient of variation of the melt viscosity during an arbitrary 3 minute period from 8 minutes after the start of measurement until the end of measurement.

[7] The easily recyclable absorbent article according to any one of [1] to [6] above, wherein the exterior part has a value of 5.0 or lower for the quotient of the maximum of the melt viscosity at 200 to 220°C measured using a flow tester under conditions with an extrusion load of 49 N, a starting temperature of 120°C and a temperature increase of 3°C/min, for pellets obtained by melt kneading the exterior part, divided by the maximum of the melt viscosity at 200 to 220°C measured by the same method for pellets obtained by melt kneading only the back sheet of the exterior part.

[8] A method for producing a recycled material, the method comprising the following steps:

(1) a step of at least melt kneading exterior parts separated from easily recyclable absorbent articles according to any one of [1] to [7] above, and
(2) a step of pelletizing the product of step (1).

[9] The method according to [8] above, wherein virgin raw material is further added in step (1).

[10] The method according to [9] above, wherein virgin raw material is added at 1 to 95 mass% to the recycled material in step (1).

[11] A method for producing a recycled material, the method comprising the following steps:

(i) a step of separating at least exterior parts from easily recyclable absorbent articles according to any one of [1] to [7] above,
(ii) a step of at least melt kneading the exterior parts separated in step (i) and
(iii) a step of pelletizing the product of step (ii).

[12] The method according to [8] above, wherein virgin raw material is further added in step (ii).

[13] The method according to [12] above, wherein virgin raw material is added at 1 to 95 mass% to the recycled material in step (ii).

[14] A method for producing fibers, the method comprising a step of fiberizing recycled material produced by the production method according to any one of [8] to [13] above.

[15] A method for producing a fiber product, the method comprising a step of using fibers produced by the method according to [14] above for production of a fiber product.

[16] A method for producing fibers, the method comprising the following steps:

(A) a step of at least melt kneading exterior parts separated from easily recyclable absorbent articles according to any one of [1] to [7] above, and
(B) a step of melt spinning the product of step (A).

[17] The method according to [16] above, wherein virgin raw material is further added in step (B).

[18] The method according to [17] above, wherein virgin raw material is added at 1 to 95 mass% to the recycled material in step (B).

[19] A method for producing fibers, the method comprising the following steps:

(a) a step of separating at least exterior parts from easily recyclable absorbent articles according to any one of [1] to [7] above,

(b) a step of at least melt kneading the exterior parts separated in step (a) and

(c) a step of melt spinning the product of step (b).

[20] The method according to [19] above, wherein virgin raw material is further added in step (b).

[21] The method according to [20] above, wherein virgin raw material is added at 1 to 95 mass% to the recycled material in step (b).

[ADVANTAGEOUS EFFECTS OF INVENTION]

According to the invention it is possible to provide absorbent articles whose exterior parts can be easily recycled, a method for producing recycled materials or fibers from the absorbent articles, and a method for producing fiber products from the fibers.

BRIEF DESCRIPTION OF DRAWINGS

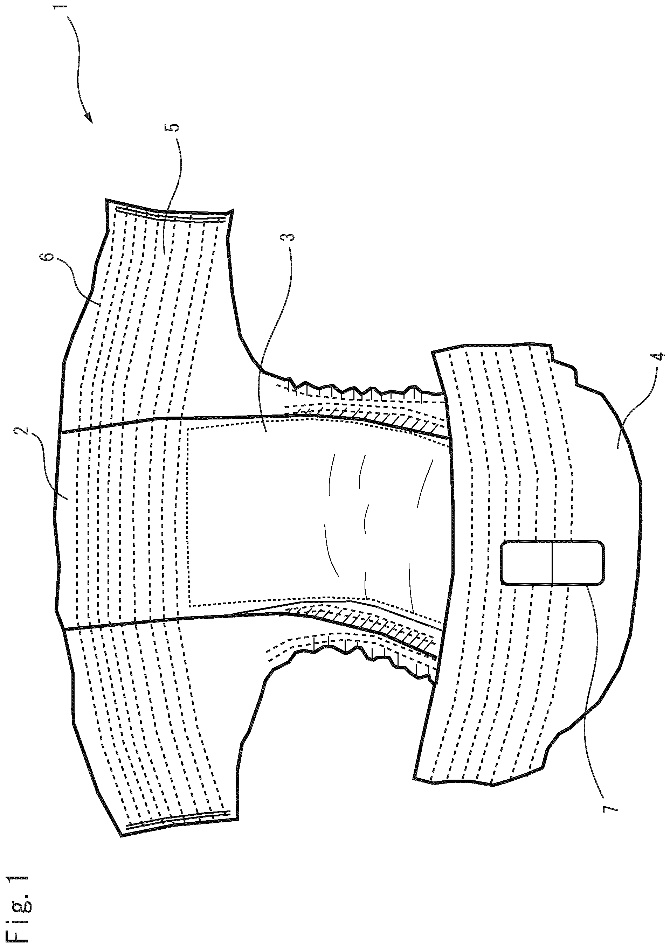

Fig. 1 is a diagram showing a construction example for an easily recyclable absorbent article according to an embodiment.

DESCRIPTION OF EMBODIMENTS

The invention will now be explained in detail by embodiments. The present invention is not limited to the embodiments described below, however, and various modifications may be implemented within the scope of the gist thereof.

The term "thermoplastic" as used herein means that the compound has a reversible property whereby it can be melted by heating at below its decomposition temperature, exhibiting plastic flow while in the molten state, and then solidifying by cooling.

The term "recycling" as used herein refers to the reuse of products that are no longer needed for daily activities and goods obtained secondary to industrial activities, as resources, or to their recovery and regeneration for effective utilization, and the term includes material recycling and chemical recycling. It also includes, for example, obtaining recycled materials from used sanitary materials or sanitary materials rejected during production due to poor quality.

One embodiment of the invention is an easily recyclable absorbent article comprising an interior part containing a front sheet and an absorbent body, and an exterior part constituting the non-interior part and containing a back sheet and a gather, wherein the back sheet includes a sheet material comprising a thermoplastic resin, and the gather includes thermoplastic elastic yarn (this will hereunder also be referred to simply as "absorbent article").

The absorbent article of this embodiment is used to absorb and retain fluids excreted from the body, which consist mainly of urine, menstrual blood and excreta. Absorbent articles include, but are not limited to, disposable diapers, sanitary napkins, incontinence pads and panty liners, for example, also including a wide variety of products used for absorption of fluids excreted from the human body.

Interior parts are the parts that comprise the wearer sides of absorbent articles, and they include front sheets and absorbent bodies. Interior parts may also include three-dimensional gathers which serve to prevent leakage.

Front sheets are liquid-permeable sheets that initially receive excreted body fluids into absorbent articles, and conventional sheet materials such as spunbond nonwoven fabrics are used.

An absorbent body is the portion that absorbs and retains body fluid excreted from the body, being disposed between a front sheet and a back sheet. Absorbent bodies used may be any conventionally known types, but they typically include pulp fibers, super-absorbent polymers (SAP) and water-absorbing sheets.

Exterior parts are portions constituting the non-interior parts, and they include back sheets and gathers. In the absorbent article of the embodiment, the back sheet includes a sheet material which comprises a thermoplastic resin, while the gather includes thermoplastic elastic yarn. In other words, it is a feature of the absorbent article of the embodiment that the main members of the exterior part all include a thermoplastic resin, and since this feature allows recycled materials or fibers to be molded by thermoforming of the exterior part as a whole, the absorbent article is easy to recycle.

**EP 4 506 401 A1**

The back sheet is a liquid-impermeable sheet that prevents leakage of body fluids retained in the absorbent body, and it comprises a sheet material that includes a thermoplastic resin. Back sheets include sheet materials such as nonwoven fabrics and synthetic resin films, or composite sheets of nonwoven fabrics and synthetic resin films. The sheet material in a back sheet may include a polyolefin resin such as polyethylene or polypropylene, a polyamide resin such as 6-nylon or 6,6-nylon, or a polyester resin such as polyethylene terephthalate (PET) or polybutylene terephthalate (PBT), and it preferably includes a polyolefin resin.

The back sheet may also include an inorganic filler or pigment. Examples of types of inorganic fillers to be included in the back sheet are calcium carbonate, calcium oxide, zeolite, amorphous aluminosilicates, clay, synthetic silica, titanium oxide, alumina, barium sulfate, aluminum sulfate and magnesium hydroxide, among which calcium carbonate, zeolite, amorphous aluminosilicates, barium sulfate, synthetic silica and magnesium hydroxide are preferred, and calcium carbonate and barium sulfate are especially preferred.

The particle size of the inorganic filler is preferably 0.1 $\mu$m to 30 $\mu$m, more preferably 1 $\mu$m to 20 $\mu$m and even more preferably 3 $\mu$m to 10 $\mu$m, from the viewpoint of discharge stability during melt kneading.

The content of the inorganic filler is preferably 20 mass% or lower, more preferably 15 mass% or lower, even more preferably 10 mass% or lower and most preferably 5 mass% or lower with respect to the mass of the exterior part, from the viewpoint of discharge stability during discharge of the molten polymer after melt kneading.

From the viewpoint of discharge stability during discharge of the molten polymer after melt kneading, the content of thermoplastic resins with an outflow start temperature of 230°C or higher in the sheet material in the back sheet is preferably 30 mass% or lower, more preferably 20 mass% or lower, even more preferably 10 mass% or lower and most preferably 5 mass% or lower with respect to the mass of the exterior part.

A gather is a stretching member with multiple folds which stretches in at least one direction, and it is provided around the waist or leg sections of an absorbent article. The gather may be formed with thermoplastic elastic yarn joining to back sheet sections, or it may be formed with thermoplastic elastic yarn joining to a separate sheet material from the back sheet, where the separate sheet material used may be a sheet material including the same thermoplastic resin as the back sheet.

The thermoplastic elastic yarn in the gather is yarn having a thermoplastic property and stretchability. Examples of thermoplastic elastic yarn include yarns comprising styrene-based, olefin-based, polyester-based and polyurethane-based thermoplastic elastomers, although elastic yarn comprising polyurethane-based thermoplastic elastomers (hereunder also referred to as "thermoplastic polyurethane elastic yarn") is preferred.

Thermoplastic elastic yarn has an outflow start temperature of preferably 160°C or higher, 170°C or higher or 180°C or higher, and preferably 220°C or lower, 215°C or lower or 210°C or lower. If the outflow start temperature is 160°C or higher the heat resistance will be sufficiently high and heat-induced yarn breakage will be less likely to occur during hot-melt adhesive coating in the absorbent article production process. If the outflow start temperature is 220°C or lower, there will be no need for thawing at excessively high temperature during recycling, thus helping to prevent progressive thermal decomposition of the polyurethane. The outflow start temperature is determined using a Shimadzu CFT-500D Flow Tester (product of Shimadzu Corp.) under conditions with a sample amount of 1.5 g, a die (nozzle) diameter of 0.5 mm and a thickness of 1.0 mm, after applying an extrusion load of 49 N and preheating for 240 seconds at an initial preset temperature of 120°C, with a uniform temperature increase rate of 3°C/min and determining the flow temperature when the polymer begins to flow out, and it is the average value for three measurements.

The thermoplastic polyurethane elastic yarn is not particularly restricted so long as it has a thermoplastic property and includes a polyurethane. The thermoplastic polyurethane elastic yarn preferably includes a polyurethane which is a polymer of a polymer polyol, a diisocyanate and an active hydrogen-containing compound that reacts with isocyanate groups.

The polymer polyol to be used for polymerization of the polyurethane is preferably a polyalkylene ether diol, polyester diol or polycarbonate diol, which are commonly used in polymerization of polyurethanes, and it is most preferably a polyalkylene ether diol with a number-average molecular weight of 900 to 3,000. Polyalkylene ether diols include those wherein the alkylene groups are tetramethylene groups, or wherein they are tetramethylene groups and straight-chain or branched alkylene groups of 1 to 8 carbon atoms. Specifically preferred are polytetramethylene ether diol, copolymerized poly(tetramethylene-neopentylene)ether diol and copolymerized poly(tetramethylene-2-methylbutylene)ether diol.

Examples of diisocyanates to be used for polymerization of the polyurethane include all aliphatic, alicyclic and aromatic diisocyanates that dissolve or are liquid under the reaction conditions, and specifically methylene-bis(4-phenylisocyanate), methylene-bis(3-methyl-4-phenylisocyanate), 2,4-tolylene diisocyanate, 2,6-tolylene diisocyanate, m- andp-xylylene diisocyanate, $\alpha,\alpha,\alpha',\alpha'$-tetramethyl-xylylene diisocyanate, m- andp-phenylene diisocyanate, 4,4'-dimethyl-1,3-xylylene diisocyanate, 1-alkylphenylene-2,4- and 2,6-diisocyanate, 3-($\alpha$-isocyanatoethyl)phenylisocyanate, 2,6-diethylphenylene-1,4-diisocyanate, diphenyl-dimethylmethane-4,4-diisocyanate, diphenyl ether-4,4'-diisocyanate, naphthylene-1,5-diisocyanate, 1,6-hexamethylene diisocyanate, methylene-bis(4-cyclohexylisocyanate), 1,3- and 1,4-cyclohexylene diisocyanate, trimethylene diisocyanate, tetramethylene diisocyanate, pentamethylene diisocyanate, hexamethylene diisocyanate and isophorone diisocyanate, with methylene-bis(4-phenylisocyanate) being especially preferred.

Examples of active hydrogen-containing compounds to be used for polymerization of the polyurethane include low-molecular-weight glycols, and specifically ethylene glycol, 1,2-propylene glycol, 1,3-propylene glycol, 2,2-dimethyl-1,3-propanediol, 1,4-butanediol, 1,3-butanediol, hexamethylene glycol, diethylene glycol, 1,10-decanediol, 1,3-dimethylolcyclohexane and 1,4-dimethylolcyclohexanehydrazine, with 1,4-butanediol being especially preferred. Alkanolamines such as monoethanolamine may also be used as active hydrogen-containing compounds.

From the viewpoint of recycling properties, the thermoplastic polyurethane elastic yarn preferably has no chemical crosslinking, and especially it preferably has no allophanate bonds with crosslinking of urethane bonds by reaction with isocyanate groups.

The thermoplastic polyurethane elastic yarn may also include additives such as stabilizers or plasticizers as necessary. Stabilizers include compounds commonly used in polyurethane resins, such as UV absorbers, antioxidants, light stabilizers, gasproof stabilizers and antistatic agents.

The exterior parts may also include hot-melt adhesives, used for adhesion between the different structural components. A hot-melt adhesive normally comprises a base polymer, a tackifier and a plasticizer. Examples of base polymers include acrylic-based, silicone-based, rubber-based and olefin-based polymers. Examples of acrylic-based polymers include (co)polymers composed mainly of vinyl monomers such as 2-ethylhexyl acrylate, butyl acrylate, ethyl acrylate, cyanoacrylate, vinyl acetate and methyl methacrylate (for example, ethylene-vinyl acetate copolymer). Examples of silicone-based polymers include polydimethylsiloxane polymers. Examples of rubber-based polymers include natural rubber, polyisoprene, styrenebutadiene copolymer (SBR), styrene-isoprene-styrene block copolymer (SIS), styrene-butadiene-styrene block copolymer (SBS), styrene-ethylene-butadiene-styrene block copolymer (SEBS) and styrene-ethylene-propylene-styrene block copolymer(SEPS).

The hot-melt adhesive content is preferably 20 mass% or lower, more preferably 15 mass% or lower, even more preferably 10 mass% or lower and most preferably 5 mass% or lower with respect to the mass of the exterior parts, from the viewpoint of reducing adhesion of the exterior parts separated from the sanitary materials during the recycling step, and thus improving handling.

The exterior parts may also include members such as fastening tape or other fastening materials, or may be modified by printing, for example, within a range that does not inhibit the recycling properties.

From the viewpoint of reducing variation in viscosity during the recycling step and improving the productivity (recycling property) of the recycled material, the coefficient of variation of the melt viscosity of the exterior part is preferably 3% or lower, more preferably 2% or lower, even more preferably 1% or lower and most preferably 0.5% or lower, as obtained by the following procedure:

> melt kneading the exterior parts to obtain pellets;
> allowing the pellets to stand for 30 minutes in the furnace of a capillary rheometer set to 230°C;
> measuring the melt viscosity at a shear rate of 1857/sec while holding at 230°C; and
> measuring the coefficient of variation of the melt viscosity during an arbitrary 3 minute period from 8 minutes after the start of measurement until the end of measurement (hereunder also referred to simply as "coefficient of variation of the melt viscosity"). A smaller coefficient of variation of the melt viscosity lowers the rate of variation in the outer shapes of the strands, improves the kneaded state in the extruder and reduces variation in discharge, thus stabilizing the form during pelletizing and improving the recycling properties. Moreover when the recycled material is used, there is less variation in viscosity during remolding such as fiberization, resulting in better moldability and improved recycling properties.

From the viewpoint of reducing variation in viscosity of the exterior parts during the recycling steps to increase productivity of the recycled material, the value obtained by dividing the maximum value of the melt viscosity at 200 to 220°C as measured using a flow tester under conditions with an extrusion load of 49 N, a starting temperature of 120°C and a temperature increase of 3°C/min, for pellets obtained by melt kneading the exterior parts, by the maximum of the melt viscosity at 200 to 220°C as measured by the same method for pellets obtained by melt kneading only the back sheets of the exterior parts (hereunder referred to as "value of the melt viscosity ratio") is preferably 5.0 or lower, more preferably 4.0 or lower and even more preferably 3.0 or lower. A smaller value for the melt viscosity ratio lowers the rate of variation in the strands, improves the kneaded state in the extruder and reduces variation in discharge, thus stabilizing the form during pelletizing and improving the recycling property. Moreover when the recycled material is used, there is less variation in viscosity during remolding such as fiberization, resulting in better moldability and improved recycling properties.

Another embodiment of the invention is a method for producing a recycled material, the method comprising the following steps:

> (1) a step of at least melt kneading exterior parts separated from easily recyclable absorbent articles as described above, and

(2) a step of pelletizing the product of step (1).

The exterior parts used for this embodiment may be separated from the absorbent articles by any means, with no particular restriction on the separation method, which may be any method known in the prior art. The absorbent articles may be pre-consumer products or post-consumer products. The exterior parts may also be washed, dried, sterilized, cut and/or pulverized before or after separation.

The exterior parts are preferably melt kneaded in a washed, dried, sterilized, cut and/or pulverized state. The method of melt kneading is not particularly restricted, and it may be a method using a screw, for example. Virgin raw material may also be added during the melt kneading to stabilize the quality of the finally obtained recycled material. During melt kneading, the structural components of the absorbent articles other than the exterior parts, including thermoplastic resins, may also be added within a range that does not inhibit the recycling properties, and for example, all of the structural components other than the absorbent body of the absorbent article may be added.

The product obtained by melt kneading is pelletized using a publicly known method such as a strand cut method, die face-cut method, flat die method, ring die method, hot cut method or screw method, for example, to yield a recycled material.

Another embodiment of the invention is a method for producing a recycled material, the method comprising the following steps:

(i) a step of separating at least exterior parts from easily recyclable absorbent articles as described above,
(ii) a step of at least melt kneading the exterior parts separated in step (i) and
(iii) a step of pelletizing the product of step (ii).

The method for separating the exterior parts from absorbent articles according to this embodiment may be any method known in the prior art without any particular limitations. The absorbent articles may be pre-consumer products or post-consumer products. The exterior parts may also be washed, dried, sterilized, cut and/or pulverized before or after separation.

The exterior parts are preferably melt kneaded in a washed, dried, sterilized, cut and/or pulverized state. The method of melt kneading is not particularly restricted, and it may be a method using a screw, for example. Virgin raw material may also be added during the melt kneading to stabilize the quality of the finally obtained recycled material. During melt kneading, the structural components of the absorbent articles other than the exterior parts, including thermoplastic resins, may also be added within a range that does not inhibit the recycling properties, and for example, all of the structural components other than the absorbent body of the absorbent article may be added.

The product obtained by melt kneading is pelletized using a publicly known method such as a strand cut method, die face-cut method, flat die method, ring die method, hot cut method or screw method, for example, to yield a recycled material.

The method for producing a recycled material may also produce strands during the pelletizing step. When strands are produced, the distance between strand discharge holes is preferably 5 mm or greater, more preferably 7 mm or greater, even more preferably 10 mm or greater and most preferably 15 mm or greater, from the viewpoint of preventing fusion or adhesion between the strands due to fluctuation or disordering of the strands. The lower limit for the outer diameters of the strands is preferably 2 mm or greater, more preferably 4 mm or greater, even more preferably 6 mm or greater and most preferably 8 mm or greater. The upper limit for the outer shapes is preferably 20 mm or less, more preferably 15 mm or less, even more preferably 12 mm or less and most preferably 10 mm or less.

The method for producing a recycled material may also include carrying out water-cooling during the pelletizing step. When water-cooling is carried out, the water temperature is preferably kept to 50°C or lower, more preferably 40°C or lower, even more preferably 30°C or lower and most preferably 20°C or lower, from the viewpoint of lowering adhesion between the polymer discharged after melt kneading and improving the cutting property for pelletizing.

When water-cooling is carried out, a water-cooling tank may be provided after the melt kneading. When a water-cooling tank is provided, the height fluctuation of the water surface is preferably 100 mm or less, more preferably 80 mm or less, even more preferably 50 mm or less and most preferably 30 mm or less, from the viewpoint of preventing fusion or adhesion between the strands due to fluctuation or disordering of the strands. When a water-cooling tank is provided, the height from the strand discharge holes to the water surface of the water-cooling tank is also preferably 500 mm or less, more preferably 300 mm or less, even more preferably 100 mm or less and most preferably 50 mm or less, from the viewpoint of preventing fusion or adhesion between the strands due to fluctuation or disordering of the strands.

In the method for producing a recycled material, the water content just before cutting of the strands is preferably 20 mass% or lower, more preferably 10 mass% or lower, even more preferably 5 mass% or lower and most preferably 3 mass% or lower, from the viewpoint of reducing stickiness of the strands during the pelletizing step.

The water content is measured by the following procedure.

Approximately 100 g of a strand is sampled just before entering the pelletizer, and the mass is measured.

The sampled strand is placed in a vacuum dryer set to a furnace temperature of 100°C and held for 12 hours in a vacuum state produced using a vacuum pump, to obtain a dried strand.

The dried strand mass is measured and the water content is determined by the following formula:

Water content (mass%) = {(Mass before drying (g) - mass after drying (g))/mass after drying (g)} $\times$ 100.

When the recycled material produced by this method is to be bagged, the pellet temperature during bagging is preferably 60°C or lower, more preferably 50°C or lower, even more preferably 40°C or lower and most preferably 30°C or lower, from the viewpoint of preventing aggregation of the recycled material.

The recycled material produced by this method can be suitably used in products such as automobile parts, household appliance parts, packaging films, food containers, caps, trays, containers, pallets, costume boxes, fibers, medical equipment, daily commodities and garbage containers.

Another embodiment of the invention is a method for producing fibers, the method comprising a step of fiberizing a recycled material produced by the method described above. The fiberizing method used may be based on any publicly known technology, such as wet spinning, dry spinning or melt spinning.

Yet another embodiment of the invention is a method for producing a fiber product using fibers produced by the method described above. The term "fiber product" used here refers to a fiber-containing product, and specific examples include woven fabrics, knitted fabrics, nonwoven fabrics, clothing, bedding supplies, interior goods, household goods, fishing nets, ropes, outdoor supplies, industrial materials, reinforcing materials for plastics and concrete, and absorbent articles. Producing an absorbent article by the method of this embodiment achieves what is known as "horizontal recycling".

Another embodiment of the invention is a method for producing fibers, the method comprising the following steps:

    (A) a step of at least melt kneading exterior parts separated from easily recyclable absorbent articles as described above, and
    (B) a step of melt spinning the product of step (A).

The exterior parts used for this embodiment may be separated from the absorbent articles by any means, with no particular restriction on the separation method, which may be any method known in the prior art. The absorbent articles may be pre-consumer products or post-consumer products. The exterior parts may also be washed, dried, sterilized, cut and/or pulverized before or after separation.

The exterior parts are preferably melt kneaded in a washed, dried, sterilized, cut and/or pulverized state. The method of melt kneading is not particularly restricted, and it may be a method using a screw, for example. Virgin raw material may also be added during melt kneading in order to stabilize spinning during the spinning step and stabilize the quality of the finally obtained fibers. During melt kneading, the structural components of the absorbent articles other than the exterior parts, including thermoplastic resins, may also be added within a range that does not inhibit the recycling properties, and for example, all of the structural components other than the absorbent body of the absorbent article may be added.

The product obtained by melt kneading is provided directly to melt spinning without pelletizing, to yield fibers. The method of melt spinning may be any method known in the prior art.

The fibers produced in this manner can be suitably used in clothing, bedding supplies, interior goods, household goods, fishing nets, ropes, outdoor supplies, industrial materials, or reinforcing materials for plastics and concrete.

Yet another embodiment of the invention is a method for producing fibers, the method comprising the following steps:

    (a) a step of separating at least exterior parts from easily recyclable absorbent articles as described above,
    (b) a step of at least melt kneading the exterior parts separated in step (a) and
    (c) a step of melt spinning the product of step (b).

The method for separating the exterior parts from absorbent articles according to this embodiment may be any method known in the prior art, without any particular limitations. The absorbent articles may be pre-consumer products or post-consumer products. The exterior parts may also be washed, dried, sterilized, cut and/or pulverized before or after separation, and for example, all of the structural components other than the absorbent bodies of the absorbent articles may be added.

The exterior parts are preferably melt kneaded in a washed, dried, sterilized, cut and/or pulverized state. The method of melt kneading is not particularly restricted, and it may be a method using a screw, for example. Virgin raw material may also be added during melt kneading in order to stabilize spinning during the spinning step and stabilize the quality of the finally obtained fibers. During melt kneading, structural components of the absorbent articles other than the exterior parts, including thermoplastic resins, may also be added within a range that does not inhibit the recycling properties.

The product obtained by melt kneading is provided directly to melt spinning without pelletizing, to yield fibers. The method of melt spinning may be any method known in the prior art.

The fibers produced in this manner can be suitably used in clothing, bedding supplies, interior goods, household goods, fishing nets, ropes, outdoor supplies, industrial materials, or reinforcing materials for plastics and concrete.

When virgin raw material is added in the melt kneading step of the method for producing recycled materials or the method for producing fibers as described above, the addition rate is preferably 1 mass% or greater, more preferably 10 mass% or greater, even more preferably 20 mass% or greater, especially preferably 30 mass% and most preferably 40 mass%, and preferably 95 mass% or lower, more preferably 80 mass% or lower, even more preferably 70 mass% or lower, especially preferably 60 mass% or lower and most preferably 50 mass% or lower, with respect to the finally produced recycled material or fibers. If the addition rate of virgin raw material is 1 mass% or greater, the weight of the virgin raw material will push it into the raw material feeder during the melt kneading step with minimal catching or clogging, even if the shapes of the separated exterior parts are non-uniform or the bulk density is low. If the addition rate of the virgin raw material is 95 mass% or lower, the difference between the melt viscosity of the exterior parts and virgin raw material will be small, reducing fluctuation in torque during melt kneading and stabilizing both the kneading state and discharge, to obtain uniform shapes for the recycled material or fibers.

In the melt kneading step of the method for producing recycled materials or the method for producing fibers described above, it is preferred to cool the starting material, such as the separated exterior parts supplied for melt kneading, specifically to a temperature of preferably 60°C or lower, more preferably 50°C or lower, more preferably 40°C or lower and most preferably 30°C or lower. The raw material feeder of the extruder is also preferably cooled, specifically so that the temperature of the surface of the chute section is preferably 100°C or lower, more preferably 80°C or lower, even more preferably 60°C or lower and most preferably 40°C or lower. Cooling of the raw material and/or the raw material feeder of the extruder will help to prevent trouble during production caused by adhesion of the exterior parts onto the apparatus or their mutual adhesion and hardening, due to the hot-melt adhesives included in the separated exterior parts, which can interfere with drawing of the exterior parts into the raw material feeder.

A lubricant may also be added during the melt kneading step in the method for producing recycled material or the method for producing fibers described above. A lubricant may be a metal salt of a fatty acid, for example. When the lubricant is a fatty acid, the number of carbon atoms of the fatty acid is preferably 8 to 30, more preferably 11 to 28 and even more preferably 12 to 22, with specific examples including metal salts of fatty acids such as stearic acid, palmitic acid, lauric acid, behenic acid and 12-hydroxystearic acid. Examples of metals to form salts with fatty acids include alkali metals such as lithium, potassium and sodium and alkaline earth metals such as magnesium, calcium and barium. Calcium stearate is a particularly suitable fatty acid metal salt as a lubricant. The lubricant is added at preferably greater than 0 mass%, and preferably 1 mass% or lower, more preferably 0.7 mass% or lower, even more preferably 0.5 mass% and most preferably 0.3 mass% or lower, with respect to the mass of the exterior parts.

From the viewpoint of improving the ability to bite and feed the raw materials into the raw material feeder during the melt kneading step of the method for producing recycled materials or the method for producing fibers, their bulk density when loaded into the raw material feeder is preferably 0.1 g/cm$^3$ to 1.0 g/cm$^3$, more preferably 0.2 g/cm$^3$ to 0.9 g/cm$^3$, even more preferably 0.3 g/cm$^3$ to 0.8 g/cm$^3$ and most preferably 0.4 g/cm$^3$ to 0.7 g/cm$^3$.

EXAMPLES

The present invention will now be explained in more specific detail through the following Examples and Comparative Examples, with the understanding that the invention is in no way limited to the Examples.

The evaluation methods used in the Examples will now be explained.

<Coefficient of variation of melt viscosity>

A measuring sample was prepared by melt kneading and pelletizing an exterior part. Using a Rheograf20 capillary rheometer (product of Goettfert Co.), a 0.5 mm × 5.0 mm capillary die was mounted onto a barrel with a barrel diameter of 9.55 mm, and heated to a furnace temperature of 230°C. About 5 g of sample was pushed in from the top of the barrel in gradual amounts while avoiding formation of air bubbles. After loading the sample, it was allowed to stand for 30 minutes with application of a load, and measurement of the melt viscosity was initiated at a shear rate of 1857/sec while maintaining a temperature of 230°C, calculating the coefficient of variation of the melt viscosity during an arbitrary continuous 3 minutes from 8 minutes after starting until completion (when the sample discharged and no longer remained in the barrel, or when the piston was pushed to the lowest point of the barrel and the equipment was in a stopped state). The arbitrary 3 minute period is selected in a range that does not include any sudden reduction in pressure caused by trapped air bubbles, or sudden increase in pressure caused by contaminants getting caught in the capillaries. Measurement is repeated if an arbitrary continuous 3 minute period cannot be ensured due to sudden pressure fluctuation. The melt viscosity $\eta$ is determined by solving the following simultaneous equations (1) to (4). The coefficient of variation (%) is the value obtained by calculating the standard deviation and average value for the melt viscosity during the arbitrary continuous 3 minute period, and dividing the standard deviation by the average value.

$$Q = A\nu \quad (1)$$

$$\gamma = 32Q/\pi D^3 \quad (2)$$

$$\tau = PD/4\,L \quad (3)$$

$$\eta = \tau/\gamma \quad (4)$$

{In the formula, Q is the flow rate of the molten resin ($mm^3$/s), A is the piston cross-sectional area ($mm^2$), v is the piston speed (mm/s), $\gamma$ is the apparent shear rate ($s^{-1}$), D is the capillary inner diameter (mm), $\tau$ is the apparent shearing stress (Pa), P is the barrel bottom pressure (Pa), L is the capillary length (mm) and $\eta$ is the melt viscosity (Pa·sec).}

<Outflow start temperature>

The elastic yarn was cut to a suitable length to prepare a measuring sample. A Shimadzu CFT-500D Flow Tester (product of Shimadzu Corp.) is used under conditions with a sample amount of 1.5 g, a die (nozzle) diameter of 0.5 mm and a thickness of 1.0 mm, after applying an extrusion load of 49 N and preheating for 240 seconds at an initial preset temperature of 120°C, with a uniform temperature increase rate of 3°C/min, and determining the plunger stroke-temperature curve during that time. The sample gradually warms as a uniform temperature increase rate is approached, causing the polymer to begin to flow out. The temperature at the point where the polymer begins to flow out is recorded as the outflow start temperature. The measurement is performed 3 times and the average temperature is recorded as the outflow start temperature.

<Value of melt viscosity ratio>

The exterior parts, and only the back sheets of the exterior parts, were each melt kneaded and pelletized to prepare separate measuring samples, and provided for the following measurement. Using a CFT-500D Shimadzu flow tester (product of Shimadzu Corp.), preheating was carried out for 240 seconds at an initial preset temperature of 120°C with a 49 N extrusion load applied, under conditions with a sample amount of 1.5 g, a die (nozzle) diameter of 0.5 mm and a thickness of 1.0 mm, after which the temperature was increased at a uniform rate of 3°C/min, periodically recording the degree of lowering of the piston when the melted pellets were extruded through the die hole below the cylinder (the outflow rate) and drawing a flow curve with the degree of lowering as the abscissa and time as the ordinate, from which the melt viscosity at 200 to 220°C was detected. The value of the melt viscosity ratio is the maximum value for the melt viscosity of the pellets obtained by melt kneading exterior parts divided by the maximum value for the melt viscosity of the pellets obtained by melt kneading only the back sheets of the exterior parts.

<Strand outer diameter fluctuation rate>

In the strand production step of the following Examples, an LS-9030D biaxial outer shape measuring tool by Keyence Corp. was set so as to allow measurement of the outer diameter of the strand at a location 35 mm below the discharge hole, measuring the outer diameter of the strand for 1 minute and calculating the fluctuation rate in the outer diameter of the strand by the following formula:

Outer diameter fluctuation rate (%) = {(Maximum outer diameter - minimum outer diameter)/average outer diameter} $\times$ 100

For this evaluation, a smaller fluctuation rate in the outer shape of the strand improves the kneaded state in the extruder and reduces variation in discharge, thus stabilizing the form during pelletizing and improving the recycling properties, or in other words, resulting in "easy recyclability". A fluctuation rate of lower than 100% may be considered to be a sufficiently satisfactory recycling property.

[Comparative Example 1]

Commercially available diapers were used as absorbent articles. The exterior parts of the diapers had as the back sheet a thermoplastic polypropylene nonwoven fabric laminated with a thermoplastic polyethylene film, with a gather formed by bonding of dry spun spandex to the back sheet with a styrene-based hot-melt adhesive. The exterior parts consisted of 75 mass% of the polypropylene nonwoven fabric, 16 mass% of the polyethylene film, 4 mass% of the dry spun spandex and 5 mass% of the hot-melt adhesive with respect to the total mass of the exterior parts. The dry spun spandex had an outflow start temperature of 235°C, and no thermoplasticity. The exterior parts were separated from multiple diapers by hand to obtain approximately 1.3 kg of exterior parts. The separated exterior parts were hot pressed at 150°C for 20 seconds through heat-resistant paper, cooled to room temperature, and cut to about 5 to 10 mm using a cutter, to obtain exterior part cut fragments. The exterior part cut fragments were melt kneaded at 160°C to 180°C using a twin-screw extruder and extruded as a strand into a water-cooling tank from three discharge holes with a hole distance of 5 mm, and after cooling, the strand was cut with a pelletizer to create pellets.

[Example 1]

A 2400 g portion of polytetramethylene ether diol with a number-average molecular weight of 1800, and 750.75 g of 4,4'-diphenylmethane diisocyanate, were reacted for 3 hours at 60°C while stirring under a dry nitrogen atmosphere, to obtain a polyurethane prepolymer capped at both ends with isocyanate groups. After mixing 9 g of AO-60 by Adeka as an antioxidant and 9 g of LA-36 by Adeka as an ultraviolet absorber into the reaction mixture, 150.95 g of 1,4-butanediol was added and the mixture was stirred for 15 minutes to obtain a polyurethane with a viscosity of 200 Pa•s (30°C).

It was then poured out onto a TEFLON$^R$ tray and annealed for 19 hours in a hot air oven at 110°C with the polyurethane in the tray, to obtain a polyurethane resin.

The obtained polyurethane resin was crushed into powder with a size of about 3 mm using a Model UG-280 crusher by Horai Co. After adding 0.35 parts by mass of dried ethylenebisstearamide to the polyurethane resin powder and loading the mixture into a hopper, it was melted in an extruder. After then metering and pressurizing with a gear pump installed at the head and filtering with a filter, the melt was discharged from a 60-hole nozzle with a 0.23 mm diameter at a die temperature of 210°C, with a throughput of 31 g/min. Cold air at a temperature of 16°C was blown in at a cold air speed of 0.6 m/s from a cold air chamber with a cold air length of 900 mm, striking the yarn perpendicularly. A ring-type false twisting machine set 5 m below was used for propagation of twisting with a 1400 mm location of convergence, as the distance from the spinneret to the location of twist propagation, after which a treatment agent composed mainly of polydimethylsiloxane and mineral oil was applied while winding up at a speed of 500 m/min, to obtain thermoplastic polyurethane elastic yarn with a monofilament size of 10 dtex, a total fineness of 620 dtex and an outflow start temperature of 182°C. The treatment agent application rate on the polyurethane elastic yarn was 2 parts by mass.

Pellets were obtained as recycled material in the same manner as Comparative Example 1, except that the dry spun spandex of the diapers used in Comparative Example 1 was changed to the aforementioned thermoplastic polyurethane elastic yarn.

[Example 2]

Polyurethane elastic yarn with an outflow start temperature of 220°C was obtained in the same manner as Example 1, except that the amount of 4,4'-diphenylmethane diisocyanate was 1134.47 g, the amount of 1,4-butanediol was 292.47 g and the die temperature was 230°C. Pellets were obtained as recycled material in the same manner as Comparative Example 1, except that the dry spun spandex of the diapers used in Comparative Example 1 was changed to the aforementioned thermoplastic polyurethane elastic yarn.

[Example 3]

Pellets were obtained as recycled material in the same manner as Example 1, except that polypropylene pellets as virgin raw material were added at 10 parts by mass to 90 parts by mass of the exterior part cut fragments, prior to melt kneading.

[Example 4]

Pellets were obtained as recycled material in the same manner as Example 3, except that the virgin raw material was added at 50 parts by mass to 50 parts by mass of the exterior part cut fragments.

[Example 5]

Pellets were obtained as recycled material in the same manner as Example 3, except that the virgin raw material was added at 80 parts by mass to 20 parts by mass of the exterior part cut fragments.

[Example 6]

Pellets were obtained as recycled material in the same manner as Example 3, except that the distance between the strand discharge holes was 10 mm.

[Example 7]

Pellets were obtained as recycled material in the same manner as Example 6, except that a temperature of 20 to 22°C was maintained in the water-cooling tank by flowing cooling water into the water-cooling tank.

[Example 8]

Pellets were obtained as recycled material in the same manner as Example 7, except that fluctuation in the liquid surface height was controlled to a maximum of 15 mm by placing a screen in the water-cooling tank and adjusting the locations of loading and drainage.

[Example 9]

Pellets were obtained as recycled material in the same manner as Example 8, except that the water content just before cutting the strand was kept to 5 mass% by blowing off the moisture adhering to the strand with compressed air, during the period from leaving the water-cooling tank until entering the pelletizer.

[Example 10]

Pellets were obtained as recycled material in the same manner as Example 8, except that the exterior parts were cut without pressing, obtaining a bulk density of 0.3 g/cm$^3$ for the cut fragments of the exterior parts.
Table 1 shows the measurement results for Comparative Example 1 and Examples 1 to 10.

[Table 1]

| | Comp. Example 1 | Example 1 | Example 2 | Example 3 | Example 4 | Example 5 | Example 6 | Example 7 | Example 8 | Example 9 | Example 10 |
|---|---|---|---|---|---|---|---|---|---|---|---|
| Elastic yarn outflow start temperature (°C) | 235 | 182 | 220 | 182 | 182 | 182 | 182 | 182 | 182 | 182 | 182 |
| Bulk density of raw material (exterior part cut fragments) (g/cm³) | 0.5 | 0.5 | 0.5 | 0.5 | 0.5 | 0.5 | 0.5 | 0.5 | 0.5 | 0.5 | 0.3 |
| Coefficient of variation of melt viscosity | 0.8 | 0.2 | 0.4 | 0.4 | 0.2 | 0.3 | 0.4 | 0.3 | 0.3 | 0.3 | 0.4 |
| Melt viscosity ratio value | 7 | 1 | 4 | 1 | 1 | 1.5 | 1 | 1 | 1 | 1 | 1 |
| Addition of virgin raw material with respect to recycled material (mass%) | 0 | 0 | 0 | 10 | 50 | 80 | 10 | 10 | 10 | 10 | 0 |
| Distance between strand discharge holes (mm) | 5 | 5 | 5 | 5 | 5 | 5 | 10 | 10 | 10 | 10 | 10 |
| Water-cooling tank internal temperature (°C) | 32-35 | 25-27 | 30-32 | 25-27 | 25-27 | 25-27 | 25-27 | 20-22 | 20-22 | 20-22 | 20-22 |
| Maximum fluctuation of liquid surface height (mm) | 20 | 20 | 20 | 20 | 20 | 20 | 20 | 40 | 15 | 15 | 15 |
| Water content just before strand cutting (mass%) | 10 | 10 | 10 | 10 | 10 | 10 | 10 | 10 | 10 | 5 | 10 |
| Strand outer diameter fluctuation rate (%) | 250 | 20 | 80 | 70 | 15 | 40 | 50 | 40 | 30 | 20 | 60 |

In Table 1 it is seen that with the absorbent articles of Examples 1 to 10 wherein all of the main structural components of the exterior parts contained thermoplastic resins, the rate of outer diameter fluctuation of the strands was low during creation of pellets as the recycled material, and therefore recycling was easy.

[Evaluation of spinnability of created pellets]

The pellets created in Comparative Example 1 and Examples 1 to 10 were each used for melt spinning by a publicly known method under the same conditions. Specifically, the pellets were melted with an extruder at 200 to 230°C, weighed by a gear pump mounted on the head, pressed, and filtered with a filter comprising a stack of three wire nets of 50 mesh, 200 mesh and 400 mesh, after which they were discharged from a 12-hole nozzle with a diameter of 0.4 mm at a discharge throughput of 15 g/min, at a die temperature of 220°C. Cold air at a temperature of 16°C was blown in at a cold air speed of 0.6 m/s from a cold air chamber with a cold air length of 900 mm, striking the yarn perpendicularly. The yarn was then wound up at a speed of 1000 m/min, and spinning was carried out under conditions to obtain yarn with a monofilament size of 1.3 dtex and a total fineness of 16 dtex. When the pellets created in Comparative Example 1 were used, the spinning pack pressure increased drastically at 0.3 MPa/min, with frequent yarn breakage once every 3 minutes, making it impossible to achieve stable spinning. When the pellets created in Examples 1 to 10 were used, however, the spinning pack pressure was 0.1 MPa/hr or lower, with no drastic increase in pressure and no yarn breakage during 5 hours of spinning, allowing stable spinning to be carried out. This demonstrated that the absorbent articles of Examples 1 to 10, wherein all of the main structural components of the exterior parts contained thermoplastic resins, allow easy recycling of the exterior parts.

INDUSTRIAL APPLICABILITY

An absorbent article of the invention is easy to recycle after disposal and can therefore be suitably used as an environmentally friendly absorbent article. Moreover, the method for producing recycled materials and the method for producing fibers of the invention can be suitably used as methods for obtaining recycled materials and fibers from waste absorbent articles.

REFERENCE SIGNS LIST

1 Absorbent article
2 Front sheet
3 Absorbent body
4 Back sheet
5 Gather
6 Thermoplastic elastic yarn
7 Fastening material

**Claims**

1. An easily recyclable absorbent article comprising:

   an interior part containing a front sheet and absorbent body, and
   an exterior part constituting the non-interior part and containing a back sheet and a gather, wherein the back sheet includes a sheet material comprising a thermoplastic resin, and
   the gather includes thermoplastic elastic yarn.

2. The absorbent article according to claim 1, wherein the outflow start temperature of the thermoplastic elastic yarn is 160°C to 220°C.

3. The easily recyclable absorbent article according to claim 1 or 2, wherein the thermoplastic elastic yarn is thermoplastic polyurethane yarn.

4. The easily recyclable absorbent article according to claim 1 or 2, wherein the exterior part contains a hot-melt adhesive.

5. The easily recyclable absorbent article according to claim 1 or 2, wherein the exterior part contains a thermoplastic film.

6. The easily recyclable absorbent article according to claim 1 or 2, wherein the coefficient of variation of the melt viscosity of the exterior part is 0.5 or lower, as obtained by the following procedure:

   melt kneading the exterior part to obtain pellets;
   allowing the pellets to stand for 30 minutes in the furnace of a capillary rheometer set to 230°C;
   measuring the melt viscosity at a shear rate of 1857/sec while holding at 230°C; and
   calculating the coefficient of variation of the melt viscosity during an arbitrary 3 minute period from 8 minutes after the start of measurement until the end of measurement.

7. The easily recyclable absorbent article according to claim 1 or 2, wherein the exterior part has a value of 5.0 or lower for the quotient of the maximum of the melt viscosity at 200 to 220°C measured using a flow tester under conditions with an extrusion load of 49 N, a starting temperature of 120°C and a temperature increase of 3°C/min, for pellets obtained by melt kneading the exterior part, divided by the maximum of the melt viscosity at 200 to 220°C measured by the same method for pellets obtained by melt kneading only the back sheet of the exterior part.

8. A method for producing a recycled material, the method comprising the following steps:

   (1) a step of at least melt kneading exterior parts separated from easily recyclable absorbent articles according to claim 1 or 2, and
   (2) a step of pelletizing the product of step (1).

9. The method according to claim 8, wherein virgin raw material is further added in step (1).

10. The method according to claim 9, wherein virgin raw material is added at 1 to 95 mass% to the recycled material in step (1).

11. A method for producing a recycled material, the method comprising the following steps:

    (i) a step of separating at least exterior parts from easily recyclable absorbent articles according to claim 1 or 2,
    (ii) a step of at least melt kneading the exterior parts separated in step (i) and
    (iii) a step of pelletizing the product of step (ii).

12. The method according to claim 11, wherein virgin raw material is further added in step (ii).

13. The method according to claim 12, wherein virgin raw material is added at 1 to 95 mass% to the recycled material in step (ii).

14. A method for producing fibers, the method comprising a step of fiberizing recycled material produced by the production method according to claim 8.

15. A method for producing a fiber product, wherein a fiber product is produced using fibers produced by the method according to claim 14.

16. A method for producing fibers, the method comprising the following steps:

    (A) a step of at least melt kneading exterior parts separated from easily recyclable absorbent articles according to claim 1 or 2, and
    (B) a step of melt spinning the product of step (A).

17. The method according to claim 16, wherein virgin raw material is further added in step (A).

18. The method according to claim 17, wherein virgin raw material is added at 1 to 95 mass% to the recycled material in step (A).

19. A method for producing fibers, the method comprising the following steps:

(a) a step of separating at least exterior parts from easily recyclable absorbent articles according to claim 1 or 2,
(b) a step of at least melt kneading the exterior parts separated in step (a) and
(c) a step of melt spinning the product of step (b).

**20.** The method according to claim 19, wherein virgin raw material is further added in step (b).

**21.** The method according to claim 20, wherein virgin raw material is added at 1 to 95 mass% to the recycled material in step (b).

Fig. 1

## INTERNATIONAL SEARCH REPORT

| International application No. |
|---|
| **PCT/JP2023/014079** |

### A. CLASSIFICATION OF SUBJECT MATTER

*C08J 11/06*(2006.01)i; *B29B 17/02*(2006.01)i; *A61F 13/49*(2006.01)i; *A61F 13/514*(2006.01)i
FI:  A61F13/514 100; A61F13/49 319; B29B17/02; C08J11/06 ZAB

According to International Patent Classification (IPC) or to both national classification and IPC

### B. FIELDS SEARCHED

Minimum documentation searched (classification system followed by classification symbols)

C08J11/06; B29B17/02; A61F13/49; A61F13/514

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2021-20437 A (KAO CORP) 18 February 2021 (2021-02-18)<br>paragraphs [0016]-[0039], fig. 1-2 | 1-5 |
| A | | 6-21 |
| Y | WO 2022/054811 A1 (ASAHI KASEI KABUSHIKI KAISHA) 17 March 2022 (2022-03-17)<br>paragraphs [0006], [0011]-[0030] | 1-5 |
| Y | JP 2000-14697 A (UNI CHARM CORP) 18 January 2000 (2000-01-18)<br>paragraph [0013] | 5 |
| A | JP 2017-36405 A (YOSHIOKA SANGYO KK) 16 February 2017 (2017-02-16)<br>entire text, all drawings | 6-21 |
| A | JP 2014-151254 A (FUKUOKA UNIV) 25 August 2014 (2014-08-25)<br>entire text, all drawings | 6-21 |

☐ Further documents are listed in the continuation of Box C.    ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **11 May 2023** | **23 May 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2023/014079**

| Patent document cited in search report | | | Publication date (day/month/year) | Patent family member(s) | | | Publication date (day/month/year) |
|---|---|---|---|---|---|---|---|
| JP | 2021-20437 | A | 18 February 2021 | (Family: none) | | | |
| WO | 2022/054811 | A1 | 17 March 2022 | CN | 116096949 | A | |
| JP | 2000-14697 | A | 18 January 2000 | US | 6387083 | B1 | |
| | | | | column 2, lines 47-59 | | | |
| | | | | EP | 970677 | A2 | |
| JP | 2017-36405 | A | 16 February 2017 | (Family: none) | | | |
| JP | 2014-151254 | A | 25 August 2014 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016079525 A

- JP 2018171780 A